# EUROPÄISCHE PATENTSCHRIFT

(11) **EP 3 027 533 B1**
(45) Veröffentlichungstag und Bekanntmachung des Hinweises auf die Patenterteilung: **13.06.2018**
(21) Anmeldenummer: 14745116.5
(22) Anmeldetag: 28.07.2014
(51) Int. Cl.: B65D 83/08, A61F 15/00

(54) **SPENDERBOX**
DISPENSING BOX
BOÎTE DE DISTRIBUTION

(30) Priorität: 29.07.2013 DE 102013214791
(43) Veröffentlichungstag der Anmeldung: 08.06.2016
(73) Patentinhaber: Paul Hartmann AG, 89522 Heidenheim (DE)
(72) Erfinder: FONFARA-DOERR, Astrid, 89077 Ulm (DE); MAHLER, Andreas, 89143 Blaubeuren-Asch (DE)
(74) Vertreter: DREISS Patentanwälte PartG mbB
(86) Internationale Anmeldenummer: PCT/EP2014/066193
(87) Internationale Veröffentlichungsnummer: WO 2015/014802

(56) Entgegenhaltungen:
- EP-A2- 0 290 032
- WO-A1-94/08541
- DE-U1- 9 405 120
- US-A- 4 807 753

## Beschreibung

Die Erfindung betrifft eine Spenderbox zur Entnahme von vorproportionierten Teilstücken von ein- oder mehrbahnigem zu einer Rolle aufgewickelten bahnförmigen Packungsgut, wie Verbandstoff und / oder Verbandmaterialien, mit einem dieses aufnehmenden verschließbaren Gehäuse, mit einer an einer Gehäuseseite vorgesehenen, mittels einer um eine Schwenkachse verschwenkbaren Verschlussklappe verschließbaren Gehäuseöffnung zum Entnehmen von Packungsgut aus dem Gehäuse.

Derartige Spenderboxen sind im Stand der Technik seit langem bekannt. So zeigt beispielsweise die DE 36 28 966 A1 eine medizinische Gerätekombination mit Tupferanfeuchter, Tupferbehälter und - halterung, wobei ein Tupferspender vorgesehen ist.

Darüber hinaus ist eine gattungsgemäße Anordnung aus der DE 290 032 bekannt, bei der die Gehäuseöffnung schlitzförmig ausgebildet ist und lediglich eine geringfügig größere Höhe aufweist als die Dicke des Packungsmaterials, wobei die Gehäuseöffnung durch die Verschlussklappe verschließbar ist.

Weiterer Stand der Technik ergibt sich aus der DE 94 05 120.8, die einen Spender für Verbandmittel zeigt, sowie der WO 94/08541, die einen Spender für Gipsbinden zum Gegenstand hat.

Dabei wird die Verschlussklappe manuell nach oben verschwenkt, wobei im offenen Zustand der Entnahmeöffnung die Verschlussklappe von der betreffenden Gehäusewand nach vorne absteht und einem Zugriff des Packungsgutes insofern hinderlich sein kann.

Die Erfindung stellt sich nun die Aufgabe, eine Spenderbox der gattungsgemäßen Art zur Verfügung zu stellen, die einen leichten Zugriff auf das Packungsgut ermöglicht und zum anderen eine leichte Reinigbarkeit des Gehäuses, insbesondere auch Desinfizierbarkeit, ermöglicht.

Die Erfindung löst diese Aufgabe durch eine Spenderbox gemäß Anspruch 1, bei der in dem Gehäuse eine Blende lösbar anbringbar ist, die die Gehäuseöffnung unter Freilassung einer schlitzförmigen Entnahmeöffnung für das Packungsgut überdeckt.

Auf diese Weise kann erreicht werden, dass die Verschlussklappe, die die Gehäuseöffnung verschließt und in ihrer Größe in etwa der Gehäuseöffnung entspricht, verhältnismäßig groß ausgebildet werden kann, sodass der Zugriff auf das Packungsgut, erleichtert wird und auch die Bedienbarkeit der Verschlussklappe durch den großen Verschwenkweg vereinfacht werden kann. Gleichzeitig wird jedoch durch die Vorsehung der Blende erreicht, dass die Gehäuseöffnung bis auf eine schlitzförmige Entnahmeöffnung durch die Blende wiederum verschlossen wird und so eine staubfreie Lagerung des Packungsguts im Gehäuse realisiert wird trotz der vergleichsweise großen vorgesehenen Gehäuseöffnung und Verschlussklappe.

Das Gehäuse besteht aus zwei Gehäusehälften, vorzugsweise einer im Gebrauchszustand oberen und einer im Gebrauchszustand unteren Gehäusehälfte. Durch diese zwei Gehäusehälften lässt sich das Gehäuse problemlos in kleinere Einheiten zerlegen, die allseitig gut zuganglich sind und eine leichte Reinigung, insbesondere auch eine Wischdesinfektion, aber auch eine Reinigung, beispielsweise in einer Spülmaschine oder in einem Desinfektionsbad ermöglichen.

Besonders bevorzugt kann vorgesehen sein, dass die Verschlussklappe einen um die Verschwenkachse verschwenkbaren Teil und einen mit dem Gehäuse fest oder lösbar verbundenen festen Teil aufweist. Dabei kann besonders bevorzugt vorgesehen sein, dass der verschwenkbare Teil vorzugsweise über ein Scharnier, z. B. ein Filmscharnier, aber auch ein herkömmliches Scharnier mit einem Zapfen, um das sich die Verschwenkklappe drehen lässt, mit dem unteren Gehäuseteil, und der feste Teil vorzugsweise mit dem oberen Gehäuseteil verbunden ist.

Auch bei Vorsehung von einer einteiligen Verschlussklappe, bei der lediglich ein verschwenkbarer Teil vorgesehen ist, ist es bevorzugt, wenn die Verschlussklappe über ein Scharnier am unteren Gehäuseteil angelenkt ist, sodass ein freies Ende der Verschlussklappe in Gebrauchsposition nach oben weist und durch ein Ziehen nach unten verschwenkt werden kann. Dies bietet den Vorteil, dass dann die Verschlussklappen parallel zu einer Aufstellfläche des Gehäuses in der vollständig geöffneten Position zum Liegen kommt und so einem Zugriff auf das Packungsgut nicht im Weg ist.

Durch die Vorsehung eines festen sowie eines verschwenkbaren Teils der Verschlussklappe lässt sich die Schließkante, d. h. die Kante, an der der schwenkbare Teil mit dem festen Teil gegeneinander anliegt, besonders gut einstellen, sodass ein sicherer, und vor allem staubfreier Verschluss realisiert werden kann. Darüber hinaus kann über diese Aufteilung in einen festen und einen verschwenkbaren Teil die Größe des verschwenkbaren Teils der Verschlussklappe wunschgemäß eingestellt werden.

Je nach Gestaltung des Gehäuses, z. B. aus Kunststoff oder aus Metall sowie des entsprechenden Herstellungsverfahrens kann das Scharnier verschieden ausgebildet sein. Sofern es sich bei dem Gehäuse um ein Kunststoffgehäuse handelt, kann der verschwenkbare Teil der Verschlussklappe über ein Filmscharnier einstückig mit dem Gehäuse, insbesondere der unteren Gehäusehälfte, verbunden sein und gemeinsam mit diesem herstellt werden.

Die lösbare Festlegung von der Blende am Gehäuse betrifft dabei die Gehäusehälften. Eine Verbindung zur ein- oder mehrteiligen Verschlussklappe ist vorzugsweise nicht vorgesehen, kann aber alternativ lösbar oder unlösbar vorgesehen sein.

Definitionsgemäß soll die ein- oder mehrteilige Verschlussklappe nicht als Teil des Gehäuses verstanden werden unabhängig davon, wie Gehäuse und Verschlussklappe miteinander verbunden sind.

Die Blende kann bevorzugt formschlüssig im Gehäuse festgelegt sein, insbesondere kann sie rastend festgelegt werden. Alternativ ist jedoch auch eine kraftschlüssige lösbare Verbindung, beispielsweise mittels Schrauben, denkbar. Weiterhin ist eine magnetische Verbindung oder eine lösbare materialabschlüssige, z. B. klebende, Verbindung vorsehbar.

Eine formschlüssige, insbesondere rastende Verbindung ist jedoch bevorzugt. Hierbei können Rastelemente vorgesehen sein, die in Öffnungen oder hinter Hinterschnitte des anderen Bauteils eingreifen. Durch eine elastische Verformung des Blendenelements, das z. B. aus Kunststoff hergestellt ist, oder als Blechteil ausgebildet sein kann, kann dann ein Ver- bzw. Entrasten vorgesehen werden. Besonders bevorzugt ist vorgesehen, dass die Blende mit dem oberen Gehäuseteil verbindbar ist, wobei die Entnahmeöffnung vorzugsweise zwischen dem Gehäuse und der Blende gebildet wird. Alternativ kann auch vorgesehen sein, dass die Blende die gesamte Gehäuseöffnung überdeckt und in der Blende eine schlitzförmige Entnahmeöffnung ausgebildet ist.

Ebenfalls ist eine klemmende Verbindung, bei der die Teile zum Verbinden verformt werden und unter Spannung aneinander festlegbar sind, denkbar.

Weiterhin ist vorgesehen, dass die Entnahmeöffnung geringfügig größer ist als das Packungsgut im Querschnitt ausgebildet ist. hierdurch kann erreicht werden, dass die Öffnung ein leichtes Entnehmen des Packungsguts ermöglicht, aber gleichzeitig das Packungsgut, das sich noch im Gehäuse befindet, bestmöglich gegen Zutritt von Staub geschützt wird. Besonders bevorzugt ist dabei auch in Richtung der Flächenerstreckung des Packungsguts lediglich eine geringfügig größere Öffnung vorgesehen, sodass das Packungsgut zwar leicht aber allseitig nur mit geringem Abstand durch die Kanten der Entnahmeöffnung umfasst ist.

Die Entnahmeöffnung ist dabei insbesondere zwischen der Blende und der unteren Gehäusehälfte ausgebildet bzw. bei einem einstückigen Gehäuse im Bereich des Gehäusebodens.

Das Packungsgut kann in Rollenform in das Gehäuse aufgenommen sein, wobei auch vorgesehen sein kann, mehrere Packungsgüter, vorzugsweise in Rollenform, in das Gehäuse aufzunehmen und wobei dann alle Packungsgüter durch eine gemeinsame Entnahmeöffnung aus dem Gehäuse entnehmbar sind. Bei dem Packungsgut handelt es sich insbesondere um Verbandmaterial. Bei dem Verbandmaterial handelt es sich bevorzugt um rechteckige Tupfer, wobei die Tupfer Abschnitte eines sich in einer Längsrichtung erstreckenden Tupferbandes sind, welches in einer Querrichtung des Tupferbandes Perforationen aufweist, an welchen die Tupfer von dem Tupferband abtrennbar sind, wobei ein Ende des Tupferbandes durch wenigstens eine Entnahmeöffnung des Spenders führbar ist und in einer Entnahmerichtung aus dem Spender ziehbar ist, wobei die Entnahmerichtung gleich der Längsrichtung des Tupferbandes ist.

Bei dem bevorzugt verwendeten Tupferband handelt es sich um ein Band, das zwei parallele Tupferbahnen nebeneinander umfasst. Hierbei sind die einzelnen Tupfer sowohl in Längs- als auch in Querrichtung über kleine Stege miteinander verbunden, bzw. durch Perforationen voneinander getrennt. Das Abreißen einzelner Tupfer ist sowohl bei einer einbahnigen Verbandrolle als auch bei einer zweibahnigen Verbandrolle möglich.

Ziel der Vorrichtung ist es, eine hygienisch einwandfreie Entnahme einzelner Tupfer zu ermöglichen. Hierbei sollte vermieden werden, dass eine erste Hand am zu entnehmenden Tupfer zieht, während die andere Hand das restliche Verbandmaterial festhält. Die Rolle der anderen Hand wird bei der vorliegenden Spenderbox vorzugsweise von dem unteren Ende der Blende übernommen.

Um zu verhindern, dass beim Entnehmen des Packungsgutes ein Kontakt der Person, die das Packungsgut entnimmt, mit dem noch nicht zu entnehmenden Packungsgut erfolgt, indem z. B. ein Teil des Packungsgutes von dem übrigen Packungsgut abgerissen werden soll und hierzu das übrige Packungsgut durch die Person, die es entnimmt, festgehalten werden muss, kann vorgesehen sein, dass die Blende an ihrer der Entnahmeöffnung zugewandten Seite ein elastisch verformbares Fixierelement aufweist, das durch die Person, die das Packungsgut aus der Spenderbox entnimmt, gegen das in der Spenderbox verbleibende Packungsgut gedrückt werden kann, so dass das Packungsgut gegen die Unterseite der Spenderbox gedrückt wird, wobei hierzu vorgesehen sein kann, dass die Spenderbox an ihrer Unterseite einen zugeordneten Bereichs des Bodens aufweist, der mit den elastisch verformbaren Fixierelement der Blende zusammenwirkt, so dass hierdurch ein Festhalten und damit ein Gegenhalten beim Abreißen eines Stücks des Packungsguts von den übrigen Packungsgut erfolgen kann, ohne das übrige Packungsgut durch Kontakt zu kontaminieren. Dabei kann vorgesehen sein, dass das Fixierelement als Federlasche ausgebildet ist.

Zur leichteren Öffenbarkeit der Verschlussklappe kann vorgesehen sein, dass diese in ihrem dem Scharnier abgewandten Bereich einen Griff aufweist. Der Griff kann dabei aus der Fläche der Verschlussklappe z. B. rechtwinklig vorstehend ausgebildet sein. Alternative können jedoch auch Griffmulden oder Grifföffnungen vorgesehen sein. Hierbei sind zum einen die Grundsätze der Ergonomie, aber auch die Reinigbarkeit ein maßgebendes Kriterium.

Besonders bevorzugt kann vorgesehen sein, dass das Gehäuse quaderförmig ausgebildet ist, wobei unter quaderförmig hier nicht nur streng quaderförmige Gehäuse, sondern auch solche mit gewölbten oder gekrümmten Seitenflächen verstanden sein sollen. Die Krümmung kann dabei über die gesamte Fläche gleichmäßig verlaufen, es sind jedoch auch Gestaltungen denkbar, bei der einzelne Flächen von gekrümmten in plane Gestaltungen übergehen und insbesondere soll unter einer quaderförmigen Form auch eine solche Form verstanden sein, bei der die Winkel alle oder teilweise nicht streng 90 Grad sind.

Besonders bevorzugt ist ebenfalls, wenn die Öffnung an einer Gehäuseschmalseite angeordnet ist und dabei bevorzugt an einer der Seiten, die im Wesentlichen vertikal verlaufen. Besonders bevorzugt nimmt die Verschlussklappe ein Drittel, vorzugsweise 40 % und weiter vorzugsweise 50 % der Seitenfläche ein, an der sie angeordnet ist. Die untere Kante der Gehäuseöffnung liegt bevorzugt auf der Höhe der Bodenfläche des Gehäuses oder geringfügig darüber.

Dir Erfindung wird im Folgenden anhand einer Zeichnung näher erläutert.

Weitere Vorteile und Merkmale der Erfindung ergeben aus den übrigen Anmeldungsunterlagen.

Dabei zeigen:
Figur 1 eine Spenderbox in einer perspektivischen Darstellung;
Figur 2 eine Seitenfläche der Spenderbox mit Verschlussklappe;
Figur 3 einen Schnitt durch die Spenderbox;
Figur 4 eine Blende in einer perspektivischen Darstellung;
Figur 5 ein Gehäuseoberteil von unten;
Figur 6 ein bevorzugtes Packungsgut.

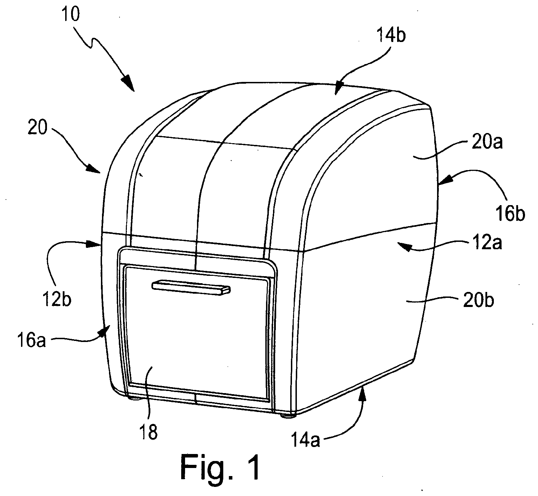

Figur 1 zeigt eine Spenderbox, die in der Gesamtheit mit den Bezugszeichen 10 versehen ist, wobei die Spenderbox 10 eine im Wesentlichen quaderförmige Form aufweist, mit zwei im Wesentlichen vertikal verlaufenden breiten Seitenflächen 12a und 12b, einer planen Unterseite 14a sowie einer gewölbten Oberseite 14b und zwei Schmalseiten 16a und 16b, die ebenfalls eine Wölbung aufweisen. An der Schmalseite 16a ist eine Verschlussklappe 18 vorgesehen. Die Spenderbox 10 umfasst dabei ein Gehäuse 20, das aus zwei Gehäusehälften, nämlich einer oberen Gehäusehälfte 20a und einer unteren Gehäusehälfte 20b gebildet ist, die form-, material- oder kraftschlüssig miteinander verbunden sein können, wobei hier eine formschlüssige, insbesondere verrastende Festlegung der beiden Teile miteinander vorgesehen ist. Die Gehäusehälften 20a, 20b bestehen aus Kunststoff.

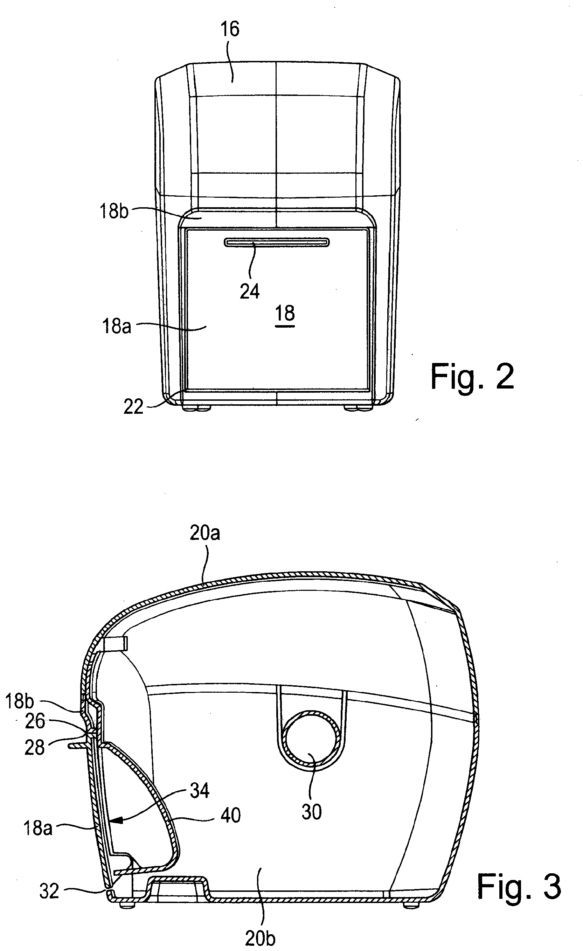

Figur 2 zeigt eine Draufsicht auf die Seite 16a, wobei diese Seitenfläche 16a insbesondere durch die Verschlussklappe 18 dominiert wird, die beinahe 50 % der gesamten Seitenfläche einnimmt. Die Verschlussklappe 18 ist um eine Verschwenkachse 22, die parallel zur unteren Kante der Verschlussklappe 18 läuft, verschwenkbar mit der unteren Gehäusehälfte 20 b verbunden. Darüber hinaus weist die Verschlussklappe einen Griff 24 auf, der aus der Fläche der Verschlussklappe 18 vorsteht und mit den Fingern einer Hand ergriffen werden kann sodass die Verschlussklappe um die Verschwenkachse 22 nach unten verschwenkt werden kann, bis sie im Wesentlichen parallel zur Bodenfläche 14a verläuft. Hierdurch erfolgt bei einem Zugriff auf das Packungsgut, das im Gehäuse 20 enthalten ist, keine Störung des Zugriffs durch die Verschlussklappe 18.

Die Verschlussklappe 18 weist dabei zwei Anteile auf, nämlich einen festen Anteil 18b sowie einen verschwenkbaren Anteil 18a. Der feste Anteil ist, wie sich insbesondere Figur 3 entnehmen lässt, mit der oberen Gehäusehälfte 20a vorzugsweise fest verbunden und bietet ein Gegenlager mit seiner Kante 26 für die Schließkante 28 des verschwenkbaren Teils 18a der Verschwenkklappe 18.

In dem Gehäuse 20 und hier verbunden mit dem unteren Gehäuseteil 20b ist eine Achse 30 vorgesehen, auf die ein rollenförmiges Packungsgut aufgeschoben und dort gehalten werden kann. Das Packungsgut kann dann durch eine Entnahmeöffnung 32 aus dem Gehäuse 20 der Spenderbox 10 entnommen werden. Durch die große Verschlussklappe 18 wird ein einfacher Zugriff auf das Packungsgut ermöglicht und darüber hinaus auch eine gute Zugänglichkeit des Gehäuseinneren, beispielsweise für Desinfektionszwecke, z. B. ein Desinfektionsbad oder eine Wischdesinfektion.

Um gleichzeitig jedoch zu erreichen, dass auch bei geöffneter Verschlussklappe 18 das Innere des Gehäuses staubdicht oder weitestgehend staubdicht und gegen Kontamination geschützt ist, ist eine Blende 40 vorgesehen, die lösbar mit dem Gehäuse, insbesondere dem oberen Gehäuseteil 20a, verbunden ist. Dabei erfolgt eine formschlüssige Festlegung, insbesondere eine Verrastung. Auf diese Weise wird die Gehäuseöffnung 34 durch das Einsetzten der Blende 40 auf die Größe der Entnahmeöffnung 32 reduziert.

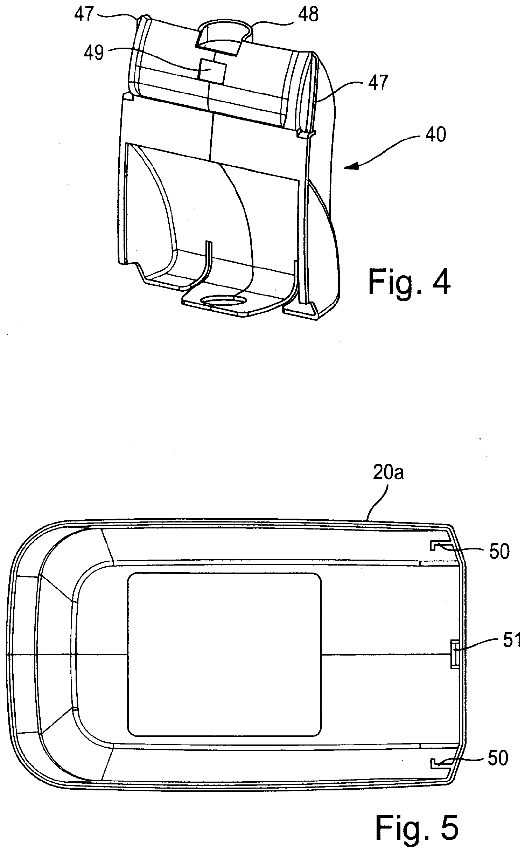

Die Blende ist in Figur 4 dargestellt, die eine perspektivische Ansicht der Blende zeigt. Hierbei weist die Blende 40 zwei Rippen 47 an ihren seitlichen Kanten auf, wobei die Rippen 47 in vertikaler Richtung verlaufen und in je einer Schiene 50 des Gehäuseoberteils 20a (Figur 5) geführt sind. Dazu wird die Blende in vertikaler Richtung (in Schienenlängsrichtung) mit den Rippen 47 in die Schienen 50 eingeschoben. Darüber hinaus ist eine Öffnung 49 vorgesehen, die durch ein Rastelement 51 des oberen Gehäuseteils 20a durchgriffen und dort lösbar festgelegt wird. Darüber hinaus weist die Blende ein Griffelement 48 auf.

Zum Lösen der Blende 40 vom Gehäuseoberteil 20a wird das Griffelement 48 ergriffen und die Elemente 51 und 49 voneinander gelöst. Danach kann die Blende durch Ziehen nach unten (in Gebrauchsposition der Spenderbox) von dem Gehäuseoberteil 20a getrennt werden.

Dabei weist die Blende an ihrer der Entnahmeöffnung zugewandten Seite ein Fixierelement 52 auf, das dem Benutzer der Spenderbox ermöglicht, ein Packungsgut durch Druck von oben auf das Element 52, das als Federlasche ausgebildet ist, so gegen einen zugeordneten Bereich des Bodens 54, der in Figur 3 zu erkennen ist, zu drücken, das ein weiteres beispielsweise Abrollen eines rollenförmigen Packungsguts in Inneren der Spenderbox verhindert wird und stattdessen das Packungsgut in der gewünschten Länge von dem als Bahnmaterial ausgebildeten Packungsgut abgetrennt werden kann. Hierzu ist eine elliptische Vertiefung 56 im Fixierelement 52 vorgesehen, um einen sicheren Druckpunkt zu erzielen.

Das Element 52 ist dabei durch zwei Schlitze 58 vom übrigen Material der Blende 40 getrennt, so dass eine federnde Gestaltung der erzielt wird.

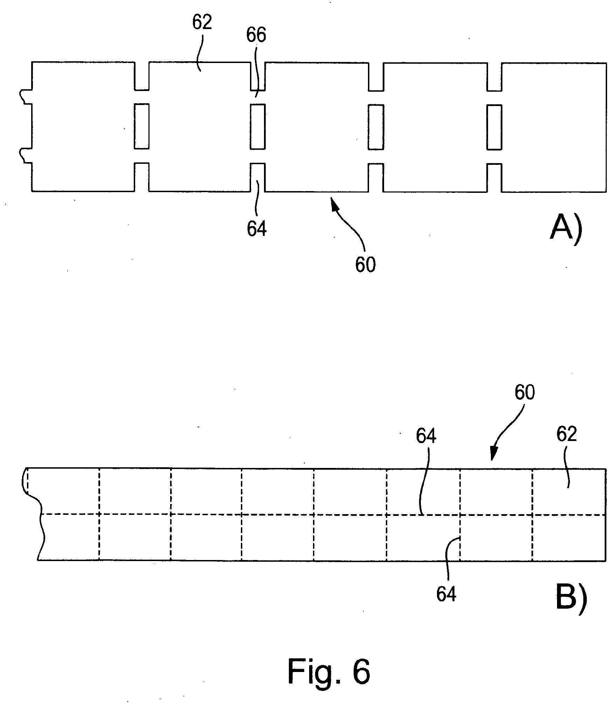

Figur 6 zeigt in Darstellung A bzw. B ein bevorzugtes Packungsgut. Das Packungsgut ist hierbei als Verbandmaterial 60 ausgebildet und besteht aus einer Vielzahl einzelner Verbandmaterialabschnitte 62, die voneinander durch eine Perforation 64 getrennt sind und lediglich über Stege 66 miteinander verbunden sind. Ein Abtrennen der einzelnen Abschnitte 62 erfolgt dann im Bereich der Perforation 64. Das Verbandmaterial 60 kann vorzugsweise als Tupfer verwendet werden. Neben der in Darstellung A gezeigten einreihigen, einbahnigen Gestaltung ist auch eine zweibahnige Gestaltung wie in Darstellung B gezeigt denkbar, bei der die einzelnen Abschnitte 62 nicht nur in Längsrichtung des Packungsgutes 60 aufeinander folgend angeordnet sind, sondern stets zwei parallele Bahnen vorgesehen sind. Die beiden Bahnen wie auch die Abschnitte 60 sind über Perforationen 64 voneinander getrennt.

Auf diese Weise kann insbesondere durch das Niederdrücken des Fixierelements 52 gegen den Bereich 54 des Bodens der Spenderbox ein Abreißen eines oder zweier Elemente 62 des Packungsguts erreicht werden, ohne dass das noch in der Spenderbox befindliche weitere Packungsgut durch Berühren mit der Hand kontaminiert wird.

## Patentansprüche

1. Spenderbox (10) zur Entnahme insbesondere von vorproportionierten Teilstücken von ein- oder mehrbahnigem zu einer Rolle aufgewickeltem Packungsgut, wie Verbandstoffe und/oder Verbandmaterialien, mit einem dieses aufnehmenden verschließbaren Gehäuse (20), mit einer an einer Gehäuseseite (16a) vorgesehenen, mittels einer um eine Schwenkachse (22) verschwenkbaren Verschlussklappe (18) verschließbaren Gehäuseöffnung (34) zur Entnahme von Packungsgut aus dem Gehäuse (20), **dadurch gekennzeichnet, dass** in dem Gehäuse (20) eine Blende (40) lösbar angebracht ist, die die Gehäuseöffnung (34) unter Freilassung einer schlitzförmigen Entnahmeöffnung (32) für das Packungsgut überdeckt, wobei das Gehäuse (20) aus zwei Gehäusehälften (20a, 20b), vorzugsweise einer im Gebrauchszustand oberen und einer unteren Hälfte besteht.

2. Spenderbox nach Anspruch 1, **dadurch gekennzeichnet dass** die Verschlusskappe (18) einen um die Verschwenkachse (22) verschwenkbaren Teil (18a) und einen mit dem Gehäuse (20) fest oder lösbar verbundenen festen Teil (18b) aufweist, wobei der verschwenkbare Teil (18a) vorzugsweise über ein Scharnier, insbesondere ein Filmscharnier mit dem unteren Gehäuseteil (20b) und der feste Teil (18b) vorzugsweise mit dem oberen Gehäuseteil (20a) verbunden ist.

3. Spenderbox nach einem der vorangehenden Ansprüche, **dadurch gekennzeichnet dass** die Blende (40) formschlüssig im Gehäuse (20) festgelegt ist, insbesondere in diesem rastend festgelegt ist.

4. Spenderbox nach einem der vorangehenden Ansprüche, **dadurch gekennzeichnet dass** die Blende (40) mit dem oberen Gehäuseteil (20a) verbunden ist.

5. Spenderbox nach einem der vorangehenden Ansprüche, **dadurch gekennzeichnet dass** die Entnahmeöffnung (32) geringfügig größer als das Packungsgut im Querschnitt ausgebildet ist.

6. Spenderbox nach einem der vorangehenden Ansprüche, **dadurch gekennzeichnet dass** das Packungsgut in Rollenform in das Gehäuse (20) aufgenommen ist.

7. Spenderbox nach einem der vorangehenden Ansprüche, **dadurch gekennzeichnet dass** mehr als ein Packungsgut in das Gehäuse (20) aufgenommen ist und alle Packungsgüter durch eine gemeinsame Entnahmeöffnung (32) aus dem Gehäuse (20) entnehmbar sind.

8. Spenderbox nach einem der vorangehenden Ansprüche, **dadurch gekennzeichnet dass** die Verschlussklappe, vorzugsweise in ihrem dem Scharnier abgewandten Bereich, einen Griff (24) aufweist.

9. Spenderbox nach einem der vorangehenden Ansprüche, **dadurch gekennzeichnet dass** das Gehäuse (20) quaderförmig, teilweise gekrümmt ist und insbesondere Seitenflächen (12, 14, 16) aufweist.

10. Spenderbox nach einem der vorangehenden Ansprüche, **dadurch gekennzeichnet dass** die Gehäuseöffnung (34) an eine Gehäuseschmalseite (16a) vorgesehen ist.

11. Spenderbox nach einem der vorangehenden Ansprüche, **dadurch gekennzeichnet dass** die Gehäuseöffnung (34) ein Drittel, vorzugsweise 40 %, vorzugsweise 50 % Öffnung der Seitenfläche (16a) des Gehäuses (20) einnimmt.

## Claims

1. A dispenser box (10) for removing in particular pre-proportioned pieces of single- or multi-ply packaged product wound up into a roll, such as bandaging fabrics and/or bandaging materials, having a closable housing (20) receiving the packaged product, having a housing opening (34), provided on one housing side (16a) and closable by means of a closure flap (18) pivotable about a pivot axis (22), for removing packaged product from the housing (20), **characterized in that** a shield (40) is detachably mounted in the housing (20) and covers the housing opening (34), leaving a slitlike removal opening (32) for the packaged product open, and the housing (20) comprises two housing halves (20a, 20b), which in the position for use are preferably one upper and one lower half.

2. The dispenser box of claim 1, **characterized in that** the closure flap (18) has one part (18a) pivotable about the pivot axis (22) and one fixed part (18b) connected fixedly or detachably to the housing (20), and the pivotable part (18a) is preferably connected via a hinge, in particular a film hinge, to the lower housing part (20b), and the fixed part (18b) is preferably connected to the upper housing part (20a).

3. The dispenser box of one of the foregoing claims, **characterized in that** the shield (40) is fixed in interlocking fashion in the housing (20), and in particular is fixed therein in detent fashion.

4. The dispenser box of one of the foregoing claims, **characterized in that** the shield (40) is connected to the upper housing part (20a).

5. The dispenser box of one of the foregoing claims, **characterized in that** the removal opening (32) is embodied as slightly larger in cross section than the packaged product.

6. The dispenser box of one of the foregoing claims, **characterized in that** the packaged product is received in roll form in the housing (20).

7. The dispenser box of one of the foregoing claims, **characterized in that** more than one packaged product is received in the housing (20), and all the packaged products can be removed from the housing (20) through the same removal opening (32).

8. The dispenser box of one of the foregoing claims, **characterized in that** the closure flap, preferably in its region facing away from the hinge, has a handle (24).

9. The dispenser box of one of the foregoing claims, **characterized in that** the housing (20) is cube-like and partly curved, and in particular has side faces (12, 14, 16) .

10. The dispenser box of one of the foregoing claims, **characterized in that** the housing opening (34) is provided on a narrow side (16a) of the housing.

11. The dispenser box of one of the foregoing claims, **characterized in that** the housing opening (34) occupies one-third, preferably 40%, preferably 50% of the opening in the side face (16a) of the housing (20).

## Revendications

1. Boîte de distribution (10) destinée à prélever en particulier des morceaux préalablement proportionnés d'un article d'emballage à une ou plusieurs bande(s) enroulé en un rouleau, tel que gaze et/ou matériel de pansement, comprenant un boîtier (20) apte à être refermé et recevant celui-ci, une ouverture de boîtier (34) qui est prévue sur un côté de boîtier (16a), est apte à être refermée au moyen d'une trappe de fermeture (18) pouvant pivoter autour d'un axe de pivotement (22), et qui sert à prendre de l'article d'emballage dans le boîtier (20), **caractérisée par le fait qu'**un obturateur (40) est monté de manière amovible à l'intérieur du boîtier (20), qui recouvre l'ouverture de boîtier (34) en laissant dégagée une ouverture de prélèvement (32) en forme de fente pour l'article d'emballage, dans laquelle ledit boîtier (20) se compose de deux moitiés de boîtier (20a, 20b), de préférence d'une moitié supérieure et d'une moitié inférieure en état d'utilisation.

2. Boîte de distribution selon la revendication 1, **caractérisée par le fait que** ladite trappe de fermeture (18) comprend une partie (18a) apte à pivoter autour de l'axe de pivotement (22) et une partie fixe (18b) qui est solidaire du boîtier (20) ou reliée de manière amovible à celui-ci, dans laquelle ladite partie (18a) apte à pivoter est reliée de préférence par une charnière, en particulier une charnière-film, à la partie de boîtier (20b) inférieure, et la partie (18b) fixe est reliée de préférence à la partie de boîtier (20a) supérieure.

3. Boîte de distribution selon l'une quelconque des revendications précédentes, **caractérisée par le fait que** ledit obturateur (40) est immobilisé à engagement positif dans le boîtier (20), en particulier est immobilisé à encliquetage dans celui-ci.

4. Boîte de distribution selon l'une quelconque des revendications précédentes, **caractérisée par le fait que** ledit obturateur (40) est relié à la partie de boîtier (20a) supérieure.

5. Boîte de distribution selon l'une quelconque des revendications précédentes, **caractérisée par le fait que** l'ouverture de prélèvement (32) est légèrement plus grande que l'article d'emballage en coupe transversale.

6. Boîte de distribution selon l'une quelconque des revendications précédentes, **caractérisée par le fait que** l'article d'emballage est logé sous forme de rouleau dans le boîtier (20).

7. Boîte de distribution selon l'une quelconque des revendications précédentes, **caractérisée par le fait que** plus d'un article d'emballage est logé dans le boîtier (20) et que l'ensemble des articles d'emballage peuvent être sortis du boîtier (20) à travers une ouverture de prélèvement (32) commune.

8. Boîte de distribution selon l'une quelconque des revendications précédentes, **caractérisée par le fait que** la trappe de fermeture présente une poignée (24), de préférence dans sa zone montrant dans la direction opposée à la charnière.

9. Boîte de distribution selon l'une quelconque des revendications précédentes, **caractérisée par le fait que** ledit boîtier (20) est en forme de parallélépipède rectangle, en partie courbé et, en particulier, présente des surfaces latérales (12, 14, 16).

10. Boîte de distribution selon l'une quelconque des revendications précédentes, **caractérisée par le fait que** l'ouverture de boîtier (34) est prévue sur un petit côté de boîtier (16a).

11. Boîte de distribution selon l'une quelconque des revendications précédentes, **caractérisée par le fait que** l'ouverture de boîtier (34) occupe un tiers, de préférence 40 %, de préférence 50 % d'ouverture de la surface latérale (16a) du boîtier (20).
